# FASCICULE DE BREVET EUROPEEN

(11) **EP 1 924 856 B1**
(45) Date de publication et mention de la délivrance du brevet: **08.07.2009**
(21) Numéro de dépôt: 06794325.8
(22) Date de dépôt: 11.08.2006
(51) Int. Cl.: G01N 33/50

(54) **MÉTHODE POUR QUANTIFIER UNE NEUROTOXINE CHOLINERGIQUE DANS UN ÉCHANTILLON**
VERFAHREN ZUR QUANTIFIZIERUNG EINES CHOLINERGEN NEUROTOXINS IN EINER PROBE
METHOD FOR QUANTIFYING A CHOLINERGIC NEUROTOXIN IN A SAMPLE

(30) Priorité: 01.09.2005 FR 0508974
(43) Date de publication de la demande: 28.05.2008
(73) Titulaire: IPSEN PHARMA, 92100 Boulogne-Billancourt (FR)
(72) Inventeur: CAMARA, José, F-75016 Paris (FR); AUGUET, Michel, F-91120 Palaiseau (FR); CHABRIER DE LASSAUNIERE, Pierre-Etienne, F-75016 Paris (FR)
(74) Mandataire: Bourgouin, André
(86) Numéro de dépôt international: PCT/FR2006/001944
(87) Numéro de publication internationale: WO 2007/026061

(56) Documents cités:
- WO-A-99/42606
- WO-A-03/013234
- US-A1- 2004 147 030
- PEARCE L B ET AL: "Pharmacologic characterization of botulinum toxin for basic science and medicine" TOXICON, vol. 35, no. 9, 1997, pages 1373-1412, XP002282904
- BEHRA MARTINE ET AL: "The use of zebrafish mutants to identify secondary target effects of acetylcholine esterase inhibitors." TOXICOLOGICAL SCIENCES, vol. 77, no. 2, février 2004 (2004-02), pages 325-333, XP002393925 ISSN: 1096-6080
- HILL ADRIAN J ET AL: "Zebrafish as a model vertebrate for investigating chemical toxicity" TOXICOLOGICAL SCIENCES, vol. 86, no. 1, juillet 2005 (2005-07), pages 6-19, XP002393924
- KRONE P H ET AL: "Use of fish liver PLHC-1 cells and zebrafish embryos in cytotoxicity assays" METHODS : A COMPANION TO METHODS IN ENZYMOLOGY, vol. 35, no. 2, février 2005 (2005-02), pages 176-187, XP004762302
- SHERIDAN R E ET AL: "Comparison of in vivo and in vitro mouse bioassays for botulinum toxin antagonists" JOURNAL OF APPLIED TOXICOLOGY, vol. 19, no. SUPPL 1, décembre 1999 (1999-12), pages S29-S33, XP002282903
- PEARCE L B ET AL: "THE MEDIAN PARALYSIS UNIT: A MORE PHARMACOLOGICALLY RELEVANT UNIT OF BIOLOGIC ACTIVITY FOR BOTULINUM TOXIN" TOXICON, vol. 33, no. 2, 1995, pages 217-227, XP000601608

## Description

La présente invention a pour objet une méthode pour déterminer la cinétique d'action d'une neurotoxine cholinergique ainsi qu'une méthode pour déterminer la quantité de neurotoxine dans un échantillon, la neurotoxine cholinergique étant une toxine clostridiale.

Les méthodes de quantification de neurotoxine cholinergique sont actuellement réalisées par la mesure d'une dose léthale DL₅₀ chez les rongeurs, et notamment chez la souris ou le rat. C'est plus particulièrement le cas de la méthode de détection et de mesure de l'activité biologique de la toxine botulique.

Or lorsqu'une telle méthode est utilisée, elle implique le sacrifice de nombreux mammifères, en particulier des souris ou des rats.

Afin de répondre aux exigences des industriels il est devenu nécessaire de trouver une méthode alternative à l'emploi des mammifères pour déterminer la dose léthale DL₅₀ des neurotoxines cholinergiques.

Aussi le problème que se propose de résoudre l'invention est de fournir une nouvelle méthode afin de déterminer la cinétique d'action d'une neurotoxine clostridiale ainsi qu'une nouvelle méthode de quantification d'une neurotoxine clostridiale.

De manière inattendue, les inventeurs ont mis en évidence qu'il est possible d'utiliser les poissons téléostéens pour déterminer la dose léthale DL₅₀ des neurotoxines clostridiales.

Dans ce but la présente invention propose une méthode pour déterminer la cinétique d'action d'une neurotoxine cholinergique comprenant les étapes suivantes :
(i) administrer la neurotoxine cholinergique à au moins un poisson téléostéen;
(ii) détecter l'activité biologique de la neurotoxine cholinergique en fonction du temps,
la neurotoxine cholinergique étant une toxine clostridiale.

La présente invention propose également une méthode pour déterminer une quantité de neurotoxine cholinergique comprenant les étapes suivantes :
(i) administrer la neurotoxine cholinergique à au moins un poisson téléostéen ;
(ii) détecter l'activité biologique de la neurotoxine cholinergique ;
(iii) comparer l'effet obtenu à l'étape (ii) à l'effet obtenu avec une substance de référence ;
(iv) déterminer la quantité de neurotoxine cholinergique,
la neurotoxine cholinergique étant une toxine clostridiale.

L'invention offre des avantages déterminants, en particulier en terme de coût économique. L'emploi de poissons téléostéens permet de significativement réduire les coûts lors de la quantification de lots de toxine botulique produits à l'échelle industrielle. En effet le dosage d'un lot de toxine botulique utilise la méthode de la DL₅₀ chez la souris et nécessite le sacrifice d'environ 100 souris.

Un autre avantage de la présente invention est que les méthodes selon l'invention sont beaucoup plus rapides à mettre en oeuvre que les méthodes connues, notamment celles qui emploient des mammifères. En effet l'évolution des effets de la neurotoxine cholinergique est plus rapidement détectable chez le poisson téléostéen que chez la souris. Dans le cas particulier où l'on observe une paralysie neuromusculaire des poissons téléostéens, cette paralysie est réversible et les poissons récupèrent une motilité normale 5 jours après administration alors qu'il faut 1 mois chez la souris pour une récupération de la motilité.

Un autre avantage de la méthode pour déterminer la cinétique d'action d'une neurotoxine cholinergique selon l'invention est de permettre d'évaluer la durée d'action de la neurotoxine, ainsi que le délai d'apparition des effets de la neurotoxine.

L'invention offre comme autre avantage que les poissons téléostéens se reproduisent très vite et en grande quantité. Il est donc possible d'obtenir un très grand nombre d'individus en un minimum de temps. Par exemple dans le cas du poisson zèbre, un adulte peut produire 100 à 200 oeufs par ponte, qui deviendront des embryons totalement formés en 48 heures et des adultes totalement formés entre 21 et 29 jours.

De plus les embryons des poissons téléostéens sont avantageusement un organisme intact, autonome à part, par rapport aux embryons de souris qui sont physiquement liés à leur mère. Cet avantage permet de tester facilement l'activité biologique d'une neurotoxine cholinergique sur des embryons.

Un autre avantage de la présente invention est qu'elle propose une méthode éthiquement plus acceptable que celles employées jusqu'à présent.

Enfin l'invention a pour avantage de pouvoir être mise en oeuvre dans toutes les industries, notamment l'industrie pharmaceutique, cosmétique ainsi que dans les domaines du traitement des effluents, de la dépollution et de l'hygiène au sens large en particulier l'hygiène au quotidien, de l'hygiène alimentaire (sécurité alimentaire) ou l'hygiène industrielle.

D'autres avantages et caractéristiques de l'invention apparaîtront clairement à la lecture de la description et des exemples donnés à titre purement illustratifs et non limitatifs qui vont suivre.

Dans ce but la présente invention propose une méthode pour déterminer la cinétique d'action d'une neurotoxine cholinergique comprenant les étapes suivantes :
(i) administrer la neurotoxine cholinergique à au moins un poisson téléostéen ;
(ii) détecter l'activité biologique de la neurotoxine cholinergique en fonction du temps,
la neurotoxine cholinergique étant une toxine clostridiale.

La présente invention propose également une méthode pour déterminer une quantité de neurotoxine cholinergique comprenant les étapes suivantes :
(i) administrer la neurotoxine cholinergique à au moins un poisson téléostéen ;
(ii) détecter l'activité biologique de la neurotoxine cholinergique ;
(iii) comparer l'effet obtenu à l'étape (ii) à l'effet obtenu avec une substance de référence ;
(iv) déterminer la quantité de neurotoxine cholinergique,
la neurotoxine cholinergique étant une toxine clostridiale.

Avantageusement la méthode selon l'invention peut permettre de déterminer une DL₅₀ d'une neurotoxine clostridiale.

Par DL₅₀ on entend au sens de la présente invention la dose léthale ou encore dose semilétale d'une substance donnée. Il s'agit de la dose qui conduit à la mort de 50 % des animaux testés d'un goupe.

La méthode selon l'invention peut également permettre de détecter l'activité biologique d'une neurotoxine clostridiale. Plus particulièrement l'activité biologique détectable selon la méthode de l'invention est une activité neuromusculaire. De préférence, il s'agit d'une paralysie musculaire. Il peut s'agir d'une paralysie des muscles lisses ou d'une paralysie des muscles striés. Cette paralysie peut entrainer la mort de l'animal.

La neurotoxine clostridiale administrée à l'étape (i) de la méthode selon l'invention peut entrainer la mort de l'animal.

La neurotoxine clostridiale administrée à l'étape (i) de la méthode selon l'invention peut être entre autre une protéine, un polypeptide, un peptide, une protéine de fusion, une protéine tronquée, une protéine chimérique, une protéine mutée ou une protéine recombinante.

Par protéine, polypeptide ou peptide on entend au sens de la présente invention, un polymère d'acides aminés, naturels ou non, lévogyre ou non, dextrogyre ou non.

Par protéine chimérique, on entend au sens de la présente invention une protéine obtenue après association de différents types de molécules, par exemple après association de lipides, de glycolipides, de peptides, de polypeptides, de protéines, de glycoprotéines, de carbohydrates, de polysaccharides, d'acides nucléiques, de polyéthylène glycol, etc.

De préférence, il s'agit d'une protéine en solution.

Selon une variante de la méthode selon l'invention la neurotoxine clostridiale administrée à l'étape (i) peut être encapsulée grâce à au moins un agent de vectorisation, comme par exemple des microsphères, des nanoparticules, des micelles ou des liposomes.

Selon une variante de la méthode selon l'invention la neurotoxine clostridiale administrée à l'étape (i) peut être associée à des agents perméabilisants tels que les salycilates, les acides gras, les chélateurs de métaux en particulier l'EDTA, des polymères cationiques, anioniques ou neutres, ou encore elle peut être associée à des formulations mucoadhésives ou transdermiques.

Selon une méthode préférentielle de l'invention la neurotoxine clostridiale est une toxine clostridiale.

De manière encore plus préférentielle, la neurotoxine clostridiale administrée à l'étape (i) est la toxine botulique, par exemple la toxine botulique de type A, B, C, D, E, F ou G.

Il peut s'agir en particulier de la toxine botulique de type A, incluant A₁, A₂ et A₃, (Dysport^{®} commercialisé par Ipsen ou Botox^{®} commercialisé par Allergan), de type B (Myobloc^{®} commercialisé par Solstice Neurosciences), de type C (incluant C₁ et C₂), de type D, de type E, de type F ou de type G.

De préférence, la neurotoxine clostridiale sera choisie parmi les toxines botuliques de type A, B et F. Encore plus préférentiellement, elle sera choisie parmi les toxines botuliques de type A et B ; en particulier, il s'agira de la toxine botulique de type A.

Tous les types de toxine botulique peuvent se lier avec une forte affinité aux neurones moteurs cholinergiques, pénétrer dans ceux-ci et bloquer le relarguage d'un neurotransmetteur l'acétylcholine dans la jonction neuromusculaire. Par exemple la toxine botulique de type A et E clive la protéine SNAP-25 (25kD) à différents sites de la protéine. Les types B, D, F et G agissent sur la protéine VAMP encore appelée synaptobrévine, chaque type de toxine clivant sa cible à différents sites. Enfin le type C1 clive à la fois la syntaxine et la protéine SNAP-25.

Par ailleurs, la toxine botulique utilisée selon la méthode de l'invention pourra se présenter sous forme d'un complexe de plusieurs composés comprenant entre autre la toxine botulique ou alors se présenter sous forme libre (i.e. libre de toute protéine la complexant). La toxine botulique peut être complexée à des protéines qui ne sont pas des toxines, on peut citer par exemple l'hémagglutinine.

L'étape (i) est réalisée en administrant la neurotoxine clostridiale à au moins un poisson téléostéen.

Le mode d'administration peut varier et de préférence la neurotoxine clostridiale est administrée par injection, plus particulièrement par injection périphérique comme par exemple par injection parentérale, sous-cutanée ou intramusculaire.

Préférentiellement, la neurotoxine clostridiale est injectée par voie intra-péritonéale.

Selon une variante de la présente invention, il est possible de prévoir que les poissons soient anesthésiés avant injection.

Il est également possible d'administrer la neurotoxine clostridiale par immersion, ingestion ou en utilisant la technique de chargement par biolistique dans laquelle des particules comprenant la neurotoxine clostridiale sont projetées dans le poisson, ses organes ou ses tissus à l'aide d'un pistolet à air comprimé.

De préférence la neurotoxine clostridiale est administrée in vivo. Selon une variante de la méthode selon l'invention, il est possible d'administrer la neurotoxine à au moins un organe ou au moins un tissu du poisson téléostéen.

Le poisson téléostéen mis en oeuvre à l'étape (i) peut-être un embryon, une larve ou un adulte. De préférence il s'agit d'un poisson âgé de 5 jours ou de 21 jours.

Par larve au sens de la présente invention, on entend un individu dont le développement se trouve entre le stade embryonnaire et le stade adulte.

Parmi les poissons téléostéens convenant pour la mise en oeuvre de la présente invention, on peut citer le poisson zèbre (Danio rerio), le poisson souffleur, le rério géant ou le médaka. Il peut également s'agir d'un poisson à phénotype sauvage, à phénotype mutant ou d'un poisson transgénique.

De préférence, il s'agit d'un poisson zèbre (ou zebrafish en anglais).

Selon une variante de la méthode selon l'invention, le ou les poissons téléostéens sont contenus dans une cupule de microtitrage d'un automate. Ceci permet avantageusement d'automatiser la méthode de quantification selon l'invention.

Il est possible de prévoir que les poissons mis en oeuvre à l'étape (i) subissent un prétraitement. Par exemple il est possible d'envisager d'immerger les poissons dans un bain contenant un antagoniste potentiel de la neurotoxine clostridiale, par exemple un anticorps anti-toxine botulique ou un inhibiteur. Il peut également être envisagé d'injecter cet antagoniste potentiel de la neurotoxine comme prétraitement avant administration de la neurotoxine.

L'étape (ii) de la méthode selon l'invention est réalisée en détectant l'activité biologique à l'aide d'un signal qui est généré. Le signal généré et détecté peut être une différence de comportement des poissons, un ralentissement de la mobilité, une diminution de la motilité, l'immobilité des poissons ou la mort des poissons.

Par motilité au sens de la présente invention, on entend le potentiel de mobilité. La motilité se compose de l'ensemble des facteurs définissant la potentialité à être mobile dans l'espace : capacités physiques, aspirations à la sédentarité ou à la mobilité, connaissances acquises. La motilité peut se transformer en déplacement, panachant ainsi différentes formes de mobilités.

De préférence, l'étape (ii) de la méthode selon l'invention est réalisée en détectant l'activité biologique à l'aide d'un analyseur d'image.

L'étape (iii) est réalisée en comparant l'effet obtenu à l'étape (ii) à l'effet obtenu avec une substance de référence.

Par substance de référence, on entend une substance étalon, ou encore une substance qui a été quantifiée par une méthode de référence. Il s'agit en général d'une neurotoxine clostridiale dont la concentration a été préalablement déterminée.

De préférence la substance de référence est une toxine clostridiale de concentration connue, plus particulièrement il s'agit de la toxine botulique, par exemple la toxine botulique de type A, B, C, D, E, F ou G.

Par toxine botulique de référence, on entend une toxine botulique qui a été quantifiée par exemple par une autre méthode que celle de l'invention ou encore par une méthode de référence. Plus particulièrement il s'agit d'une toxine botulique dont la concentration a été préalablement déterminée.

L'étape (iv) de la méthode selon l'invention est réalisée en déterminant la quantité de neurotoxine clostridiale. Cette quantité peut être de préférence exprimée en unité de toxine, 1 unité de toxine correspond à la dose qui conduit à la mort de 50 % des animaux testés.

De manière préférentielle, la méthode selon l'invention est une méthode pour déterminer la DL₅₀ d'un échantillon contenant de la toxine botulique.

Toujours de manière très préférentielle il s'agit d'une méthode pour déterminer la DL₅₀ de la toxine botulique, de préférence de la toxine botulique de type A, comprenant les étapes suivantes :
(i) injecter la toxine botulique à au moins un poisson zèbre ;
(ii) détecter l'activité biologique de la toxine botulique ;
(iii) comparer l'effet obtenu à l'étape (ii) à l'effet obtenu avec une toxine botulique de référence ;
(iv) déterminer la DL₅₀ de la toxine botulique.

Selon une autre variante très préférentielle, la méthode selon l'invention est une méthode pour déterminer la cinétique d'action de la toxine botulique comprenant les étapes suivantes :
(i) injecter la toxine botulique à au moins un poisson zèbre ;

Les exemples suivants illustrent l'invention sans en limiter la portée.

### EXEMPLES:

La toxine botulique utilisée dans ces exemples provient de chez Ipsen. Il s'agit d'un lot du Dysport^{®}.

Un contrôle négatif est réalisé en utilisant une protéine non toxique, l'albumine sérique de bovins (BSA).

### 1/ Injection des poissons zèbre :

Le Dysport^{®} est mis en solution de la manière suivante : 3 mg de Dysport® équivalant à 580 unités sont dissous dans 26 µl d'eau déionisée, ce qui aboutit à une concentration de 115 mg/ml (22 000 U/ml). Cette solution est diluée 4 fois pour obtenir une solution à 28,75 mg/ml soit 5500 U/ml.

La BSA est mise en solution à la concentration de 115 mg/ml et 28,75 mg/ml dans de l'eau déionisée.

Des poissons zèbre âgés de 5 ou de 21 jours sont injectés par voie intra-péritonéale avec le Dysport^{®} à tester :
- 10 à 20 nl de solution à tester pour les poissons âgés de 5 jours ;
- 40 à 80 nl de solution à tester pour les poissons âgés de 21 jours.

### 2/ Résultats :

### 2-1 Méthode pour déterminer une quantité de Dysport^{®} avec des poissons zèbre âgés de 5 jours :

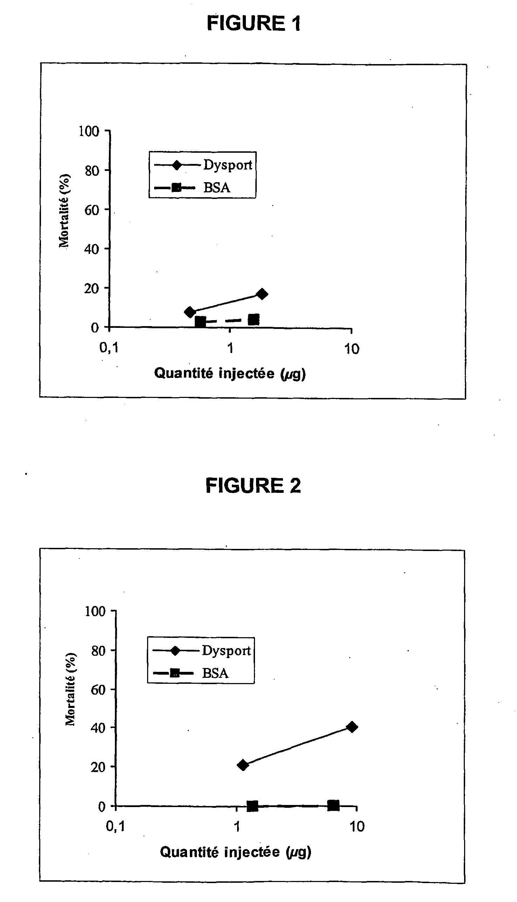

30 poissons zèbre ont reçu une injection intra-péritonéale de Dysport^{®}. Les poissons sont nourris quotidiennement et l'eau de l'aquarium est changée régulièrement. La mortalité des poissons est observée 4 jours après injection. Les résultats sont présentés dans les tableaux 1 suivants et sur la figure 1.

**Tableaux I**

| | | |
|---|---|---|
| **BSA** | Quantité injectée (µg) | |
| | 0,58 | 1,6 |
| Mortalité (%) | 3 4 | |
| **DYSPORT**^{®} | Quantité injectée (µg) | |
| | 0,46 | 1,84 |
| Mortalité (%) | 8 | 17 |

### 2-2 Méthode pour déterminer une quantité de Dysport^{®} avec des poissons zèbre âgés de 21 jours :

20 poissons zèbre ont reçu une injection intra-péritonéale de Dysport^{®}. Les poissons sont nourris quotidiennement et l'eau de l'aquarium est changée régulièrement. Les résultats sont présentés dans les tableaux II suivants et sur la figure 2.

**Tableaux II**

| | | |
|---|---|---|
| **BSA** | Quantité injectée (µg) | |
| | 1,4 | 6,55 |
| Mortalité (%) | 0 | 4 |
| | | |
| **DYSPORT**^{®} | Quantité injectée (µg) | |
| | 1,15 | 9,2 |
| Mortalité (%) | 21 | 41 |

### 2-3 conclusion:

Cette méthode permet la mise en évidence d'une activité dose-dépendante de la toxine botulique par rapport à une protéine de référence la BSA.

### 2-4 Méthode pour déterminer la cinétique du Dysport^{®} avec des poissons zèbre âgés de 21 jours :

Après 24 heures, les poissons zèbre traités avec du Dysport^{®} montrent une atteinte neuromusculaire marquée.

Après 48 heures, 20 % des poissons zèbre traités sont encore entièrement paralysés, les autres ne pouvant pas nager et leurs mouvements étant limités à de faibles mouvements, non coordonnés, de la nageoire caudale. Des effets similaires sont observés sur les poissons zèbre âgés de 5 et de 21 jours. Cependant l'administration de Dysport^{®} n'enlève pas aux poissons, leurs capacités à rester verticaux.

Après 3 jours, la mobilité des poissons est partiellement recouvrée et la récupération est totale 4 jours après administration de la neurotoxine cholinergique.

## Revendications

1. Méthode pour déterminer la cinétique d'action d'une neurotoxine cholinergique comprenant les étapes suivantes :
(i) administrer la neurotoxine cholinergique à au moins un poisson téléostéen ;
(ii) détecter l'activité biologique de la neurotoxine cholinergique en fonction du temps,
la neurotoxine cholinergique étant une toxine clostridiale.

2. Méthode pour déterminer une quantité de neurotoxine cholinergique comprenant les étapes suivantes :
(i) administrer la neurotoxine cholinergique à au moins un poisson téléostéen ;
(ii) détecter l'activité biologique de la neurotoxine cholinergique ;
(iii) comparer l'effet obtenu à l'étape (ii) à l'effet obtenu avec une substance de référence ;
(iv) déterminer la quantité de neurotoxine cholinergique,
la neurotoxine cholinergique étant une toxine clostridiale.

3. Méthode selon la revendication 1 ou 2 **caractérisée en ce que** l'activité biologique à détecter est une activité neuromusculaire, de préférence une paralysie musculaire.

4. Méthode selon la revendication 1 ou 2 **caractérisée en ce que** l'étape (i) est réalisée par injection, comme par exemple par injection parentérale, sous-cutanée ou intramusculaire.

5. Méthode selon la revendication 1 ou 2 **caractérisée en ce que** la toxine clostridiale est une protéine, un polypeptide, un peptide, une protéine de fusion, une protéine tronquée, une protéine chimérique, une protéine mutée ou une protéine recombinante.

6. Méthode selon la revendication 1 ou 2 **caractérisée en ce que** la toxine clostridiale administrée à l'étape (i) est associée à des agents perméabilisant tels que les salycilates, les acides gras ou les chélateurs de métaux en particulier l'EDTA, des polymères cationiques, anioniques ou neutres.

7. Méthode selon la revendication 1 ou 2 **caractérisée en ce que** la toxine clostridiale est la toxine botulique, par exemple la toxine botulique de type A, de type B, de type C, de type D, de type E, de type F ou de type G.

8. Méthode selon la revendication 7 **caractérisée en ce que** la toxine botulique est de type A1, A2 ou A3.

9. Méthode selon la revendication 1 ou 2 **caractérisée en ce que** le poisson téléostéen est un poisson à phénotype sauvage, à phénotype mutant ou un poisson transgénique.

10. Méthode selon la revendication 1 ou 2 **caractérisée en ce que** le poisson téléostéen est un poisson zèbre (Danio rerio), un poisson souffleur, un rério géant ou un médaka.

11. Méthode selon la revendication 1 ou 2 **caractérisée en ce que** le ou les poissons téléostéens sont contenus dans une cupule de microtitrage d'un automate.

12. Méthode selon la revendication 1 ou 2 **caractérisée en ce que** l'étape (ii) est réalisée avec un analyseur d'image.

13. Méthode selon la revendication 2 **caractérisée en ce que** la méthode permet de déterminer une DL₅₀ d'une toxine clostridiale.

## Claims

1. Method for determining the kinetics of action of a cholinergic neurotoxin, comprising the following steps:
(i) administering the cholinergic neurotoxin to at least one teleostian fish;
(ii) detecting the biological activity of the cholinergic neurotoxin as a function of time,
wherein the cholinergic neurotoxin is a clostridial toxin.

2. Method for determining a quantity of cholinergic neurotoxin comprising the following steps:
(i) administering the cholinergic neurotoxin to at least one teleostian fish;
(ii) detecting the biological activity of the cholinergic neurotoxin;
(iii) comparing the effect obtained in step (ii) to the effect obtained with a reference substance;
(iv) determining the quantity of cholinergic neurotoxin,
wherein the cholinergic neurotoxin is a clostridial toxin.

3. Method according to claim 1 or 2, **characterized in that** the biological activity to be detected is a neuromuscular activity, preferably a muscular paralysis.

4. Method according to claim 1 or 2, **characterized in that** step (i) is carried out by injection, such as for example by parenteral, subcutaneous or intramuscular injection.

5. Method according to claim 1 or 2, **characterized in that** the clostridial toxin is a protein, a polypeptide, a peptide, a fusion protein, a truncated protein, a chimeric protein, a mutated protein or a recombinant protein.

6. Method according to claim 1 or 2, **characterized in that** the clostridial toxin administered in step (i) is combined with permeabilizing agents such as salicylates, fatty acids, metal chelation agents, in particular EDTA, cationic, anionic or neutral polymers.

7. Method according to claim 1 or 2, **characterized in that** the clostridial toxin is botulinum toxin, for example botulinum toxin of type A, type B, type C, type D, type E, type F, or type G.

8. Method according to claim 7, **characterized in that** the botulinum toxin is of type A1, A2 or A3.

9. Method according to claim 1 or 2, **characterized in that** the teleostian fish is of wild type, of mutated phenotype or is a transgenic fish.

10. Method according to claim 1 or 2, **characterized in that** the teleostian fish is a zebra fish (Danio rerio), a puffer fish, a giant rerio or a medaka.

11. Method according to claim 1 or 2, **characterized in that** one or more teleostian fish are contained in a microtitration well of an automatic device.

12. Method according to claim 1 or 2, **characterized in that** step (ii) is carried out with an image analyzer.

13. Method according to claim 2, **characterized in that** the method enables determination of an LD₅₀ for a clostridial toxin.

## Patentansprüche

1. Verfahren zur Bestimmung der kinetischen Wirkung eines cholinergen Neurotoxins, umfassend die folgenden Schritte:
(i) Verabreichen des cholinergen Neurotoxins an zumindest einen Echten Knochenfisch (Teleostei);
(ii) Detektieren der biologischen Aktivität des cholinergen Neurotoxins als Funktion der Zeit,
wobei das cholinerge Neurotoxin ein Clostridientoxin ist.

2. Verfahren zur Bestimmung der Menge eines cholinergen Neurotoxins, umfassend die folgenden Schritte:
(i) Verabreichen des cholinergen Neurotoxins an zumindes einen Echten Knochenfisch (Teleostei);
(ii) Detektieren der biologischen Aktivität des cholinergen Neurotoxins;
(iii) Vergleichen des in Schritt (ii) erhaltenen Effekts mit einer Referenzsubstanz;
(iv) Bestimmen der Menge des cholinergen Neurotoxins,
wobei das cholinerge Neurotoxin ein Clostridientoxin ist.

3. Verfahren nach Anspruch 1 oder 2, **dadurch gekennzeichnet, dass** die zu detektierende biologische Aktivität eine neuromuskuläre Aktivität, vorzugsweise eine Muskellähmung, ist.

4. Verfahren nach Anspruch 1 oder 2, **dadurch gekennzeichnet, dass** Schritt (i) durch Injektion durchgeführt wird, zum Beispiel durch parenterale, subkutane, oder intramuskuläre Injektion.

5. Verfahren nach Anspruch 1 oder 2, **dadurch gekennzeichnet, dass** das Clostridientoxin ein Protein, ein Polypeptid, ein Peptid, ein Fusionsprotein, ein trunkiertes Protein, ein chimäres Protein, ein mutiertes Protein oder ein rekombinantes Protein ist.

6. Verfahren nach Anspruch 1 oder 2, **dadurch gekennzeichnet, dass** das in Schritt
(i) verabreichte Clostridientoxin mit permeabilisierenden Agenzien kombiniert wird, wie zum Beispiel mit Salizylaten, Fettsäuren, metallchelatbildenden Agenzien, insbesondere EDTA, kationischen, anionischen oder neutralen Polymeren.

7. Verfahren nach Anspruch 1 oder 2, **dadurch gekennzeichnet, dass** das Clostridientoxin Botulinumtoxin ist, zum Beispiel Botulinumtoxin vom Typ A, Typ B, Typ C, Typ D, Typ E, Typ F oder Typ G.

8. Verfahren nach Anspruch 7, **dadurch gekennzeichnet, dass** das Botulinumtoxin vom Typ A1, A2 oder A3 ist.

9. Verfahren nach Anspruch 1 oder 2, **dadurch gekennzeichnet, dass** der Echte Knochenfisch (Teleostei) einen Wildtyp oder einen mutierten Phänotyp aufweist, oder ein transgener Fisch ist.

10. Verfahren nach Anspruch 1 oder 2, **dadurch gekennzeichnet, dass** der Echte Knochenfisch (Teleostei) ein Zebrafisch (Danio rerio), ein Kugelfisch, ein Malabarkärpfling oder ein Japankärpfling ist.

11. Verfahren nach Anspruch 1 oder 2, **dadurch gekennzeichnet, dass** der oder die Echte(n) Knochenfisch(e) (Teleostei) in einer Mikrotiterplattenvertiefung für einen automatischen Apparat enthalten sind.

12. Verfahren nach Anspruch 1 oder 2, **dadurch gekennzeichnet, dass** Schritt (ii) mit einem Bildanalysesystem durchgeführt wird.

13. Verfahren nach Anspruch 2, **dadurch gekennzeichnet, dass** das Verfahren die Bestimmung einer LD₅₀ eines Clostridientoxins erlaubt.
